Europäisches Patentamt

European Patent Office

Office européen des brevets

(11) Veröffentlichungsnummer: **0 157 862 B1**

(12) **EUROPÄISCHE PATENTSCHRIFT**
veröffentlicht nach Art. 158 Abs. 3
EPÜ

(45) Veröffentlichungstag der Patentschrift: 20.03.91

(51) Int. Cl.5: **A61M 23/00, A61M 25/01**

(21) Anmeldenummer: 84903748.6

(22) Anmeldetag: 04.10.84

(86) Internationale Anmeldenummer:
PCT/EP84/00305

(87) Internationale Veröffentlichungsnummer:
WO 85/01444 (11.04.85 85/09)

(54) FÜHRUNGSMANDRIN FÜR KATHETER UND DERGLEICHEN INSTRUMENT UND VERFAHREN ZU SEINER HERSTELLUNG.

(30) Priorität: 04.10.83 DE 3336045
04.10.83 DE 3336046

(43) Veröffentlichungstag der Anmeldung:
16.10.85 Patentblatt 85/42

(45) Bekanntmachung des Hinweises auf die
Patenterteilung:
20.03.91 Patentblatt 91/12

(84) Benannte Vertragsstaaten:
AT BE CH DE FR GB LI LU NL SE

(56) Entgegenhaltungen:
US-A- 3 452 742
US-A- 3 789 841
US-A- 3 973 556

"Neuer zentraler Venenkatheter: arteriell und
venös einsetzbar" M. Hubmar et al. Notfall-
medizin 9(1983) Seiten 573-581.

(73) Patentinhaber: MÄRZ, Peter, Dr.
Frauenschuhstrasse 31
W-8122 Penzberg(DE)

Patentinhaber: POSTEL, Jürgen, Dr.
Hahnemannstrasse 2
W-8000 München 50(DE)

(72) Erfinder: MÄRZ, Peter, Dr.
Frauenschuhstrasse 31
W-8122 Penzberg(DE)
Erfinder: POSTEL, Jürgen, Dr.
Hahnemannstrasse 2
W-8000 München 50(DE)

(74) Vertreter: Hansmann, Axel et al
Patentanwälte HANSMANN & VOGESER
Albert-Rosshaupter-Strasse 65
W-8000 München 70(DE)

## Beschreibung

Die Erfindung betrifft einen Führungsmandrin entsprechend dem Oberbegriff des Anspruchs 1 sowie ein Verfahren zu seiner Herstellung.

In der Medizin ist es in den verschiedensten Bereichen oft notwendig, aus diagnostischen oder therapeutischen Gründen in Organe oder Hohlräume des Körpers einen dünnen Kunststoffkatheter einzubringen, über den dann entweder Medikamente zugeführt werden oder durch den diagnostische Messungen vorgenommen werden.

In der Angiographie, also der röntgenologischen Gefäßdarstellung von Arterien, wird eine Arterie mit einer scharfen Punktionsnadel punktiert. Durch diese Punktionsnadel wird ein Führungsmandrin in ein Gefäß eingeführt. Nach Entfernen der scharfen Punktionsnadel wird über den Führungsmandrin ein dünner, flexibler Kunststoffkatheter in das Gefäß eingelegt. über diesen Kunststoffkatheter kann dann ein Röntgenkontrastmittel injiziert werden. Das gleiche Verfahren wird auch zur kontinuierlichen Blutdruckmessung angewandt. Dabei wird an den Katheter ein Druckmeßgerät angeschlossen. Da die Lage des Führungsmandrins bei der Anwendung durch Vor- und Zurückschieben korrigiert werden muß, ist es erforderlich, daß der Führungsmandrin aus einem stabilen Material, normalerweise Metall besteht, das beim Zurückziehen durch die scharfe Punktionskanüle nicht abgeschert werden darf. Die Arterien werden soweit wie möglich tangential, aber auch mehr oder weniger in einem steilen Winkel, evtl. sogar senkrecht zum Gefäßverlauf, punktiert. Es ist deshalb erforderlich, daß der Mandrin über eine ausreichende Flexibilität verfügt, so daß kleine Biegeradien möglich sind.

Ein weiteres Problem ist die Punktion des Periduralraumes. Es handelt sich dabei um einen sehr schmalen (ca. 1 mm) Hohlraum, der im Wirbelkanal liegt und das Rückenmark umgibt. Zum Beispiel zur Anästhesie muß in diesem Periduralraum ein dünner Kunststoffkatheter eingeführt werden. Nach den bisher bekannten Methoden wird der Periduralraum mit einer relativ dicken Nadel punktiert und durch diese Nadel wird ein dünner, sehr flexibler Kunststoffkatheter vorgeschoben. Die hohe Flexibilität des Katheters ist erforderlich, um den nahezu rechten Winkel am Austritt der Punktionskanüle zu überwinden. Dadurch ergibt sich jedoch das Problem, daß der Katheter in der Regel nur wenige Zentimeter vorgeschoben werden kann, da er über keine ausreichende Stabilität verfügt. Der Katheter neigt dazu, sich im Periduralraum aufzurollen. Die Punktion muß deshalb immer in Höhe des Rückenmarksegments erfolgen, das anästhesiert werden soll. Eine Punktion in den unteren Bereichen der Wirbelsäule mit anschließendem Hochschieben des Katheters über längere Strecken wäre wünschenswert, da eine Punktion in den unteren Abschnitten weit ungefährlicher ist als in den höheren Abschnitten der Wirbelsäule. Dies ist nur durch eine sog. indirekte Technik möglich, d.h., daß zuerst durch die Punktionskanüle ein relativ stabiler Führungsmandrin hochgeschoben wird, der über eine ausreichende Flexibilität verfügt, um den rechten Winkel am Austritt der Funktionskanüle zu überwinden. über diesen Mandrin kann dann ein dünner Kunststoffkatheter vorgeschoben werden. Oder der Katheter wird vor der Punktion innen mit dem Mandrin versteift, der enge Biegeradien überwinden kann und eine ausreichende Stabilität hat.

Außerdem ist es häufig erforderlich, gewundene Hohlorgane wie z.B. Gallengänge oder Drüsenausführgänge zu sondieren, um z.B. ihre Durchgängigkeit zu prüfen oder einen Katheter einzulegen, durch den dann ein Kontrastmittel oder Medikamente eingebracht werden können.

Zum Anlegen von Venenkathetern, über die Infusionen verabreicht oder Druckmessungen durchgeführt werden können, sind ebenfalls derartige Führungsmandrine in Gebrauch.

Aus den vorstehend beschriebenen Anwendungsbereichen ergeben sich die folgenden Anforderungen, die ein idealer, universel einsetzbarer Führungsmandrin erfüllen muß:

1. Der Führungsmandrin sollte eine hohe Flexibilität haben, um geringe Biegeradien zu ermöglichen;

2. der Führungsmandrin sollte eine ausreichende Stabilität haben, um das Vorschieben zu ermöglichen;

3. der Führungsmandrin sollte aus einem Material bestehen, das auch bei scharfen Punktionskanülen verwendet werden kann, so daß der Mandrin beim zurückziehen über eine Punktionskanüle nicht abgeschert werden kann;

4. der Führungsmandrin sollte nicht knickempfindlich sein;

5. der Führungsmandrin sollte als billiges Massenprodukt herstellbar sein, da er in der Regel aus hygienischen Gründen nur einmal verwendet wird.

Ein Führungsmandrin entsprechend der eingangs genannten Gattung ist als sog. Seldinger-Spirale bekannt. Diese besteht aus einer Drahtseele, die von einem spiralförmig gewickelten Draht in Form einer Spiralfeder umgeben ist. Das proximale Ende der Drahtseele ist gegenüber dem proximalen Ende der Spiralfeder etwas zurückgesetzt. Die Spiralfeder kann außen mit Kunststoff beschichtet sein. Bei diesem Führungsmandrin bildet der überstehende Teil der Spiralfeder eine flexible Spitze, die sich den Biegungen eines punktierten Hohlorganes anpassen kann. Diese Mandrin hat eine ausreichende Flexibilität, jedoch

ist er z.B. bei der Periduralpunktion nicht einsetzbar, da bei sehr kleinen Biegeradien nur die Spitze des Mandrins entsprechend verformbar ist, nicht aber der sich anschließende Teil, der mit der Drahtseele versteift ist. Außerdem knickt die Spitze beim Verschieben im Periduralraum leicht ab, da sie infolge der steil zur Achse verlaufenden Spiralwindungen gegen seitliche Kräfte anfällig ist. Ein weiterer Nachteil liegt darin, daß beim Auftreten eines Knicks der Mandrin nicht mehr verwendbar ist, da solch ein Knick eine bleibende Verformung der Spiralfeder ist. Ein weiterer, erheblicher Nachteil liegt in den hohen Anschaffungskosten eines solchen Mandrins, die auf die hohen Herstellungskosten zurückzuführen sind.

Mit der vorstehend erläuterten Seldinger-Spirale befaßt sich auch die Literaturstelle "Notfall-Medizin" 9 (1983), Seiten 573 ff, (Perimed-Verlag). Diese Literaturstelle beschreibt auch einen weiteren Mandrin in Form eines plastikummantelten Stahldrahtes mit mehreren dünnen verdrillten Einzeldrähten, wobei durch Vorbiegung des Mandrins Anomalien oder Fehllagen leichter korregierbar sein sollen (Seite 576, oberer Absatz und Abb. 7). Bei dem hier gezeigten und in seiner Funktion erläuterten Mandrin sind die verdrillten Einzeldrähte mit hoher Steigung (also beispielsweise mindestes 5 : 1)um die Drahtseele gewickelt. Infolge der großen Steigung verlaufen die Aussendrähte mit einer erheblichen Komponente in Längsrichtung des Mandrins, also in Schieberichtung. Damit sollen geringe Reibungs- und Adhesionswerte zwischen Mandrin und Katheter erreicht werden. Dabei soll aufgrund der zwischen jeweils zwei Aussendrähten verlaufenden schraubenförmigen Furche eine zylindrische Oberfläche des Mandrins vermieden und die Oberfläche des Mandrins vielmehr von den Aussenseiten der einzelnen Aussendrähte gebildet werden. Gemäß dem ausdrücklichen Hinweis auf eine Vorformung oder Vorbiegung in eine stabile Lage, hat man also eine Ausführung in Federstahldraht, wie sie gemäß den nachstehenden Ausführungen bei mit geringer Steigung schraubenlinienförmig gewickelten Mandrins bekanntgeworden ist, nicht für richtig gehalten, sondern statt dessen die Vorformbarkeit vorgesehen, wobei der aus mehreren dünnen verdrillten Einzeldrähten bestehende Mandrin auch sehr flexibel sein soll. Eine solche Ausgestaltung mag in Anbetracht der vorgesehenen Flexibilität und Vorformbarkeit des Materials der Einzeldrähte in seiner Anwendung geringes Verletzungsrisiko durch steil und starr nach vorne stehenden Drahtenden erwarten lassen und somit anwendungsgünstig erscheinen. (Eine Beschreibung und Darstellung des vorliegend erläuterten Mandrins gemäß der Literaturstelle "Notfall-Medizin" findet sich auch in der erst nachveröffentlichten, aber älteren EP 0 132 694 und deren deutscher Grundlage DE-A-33 27 779).

Die Anwendungserfordernisse für derartige Führungsmandrins sind jedoch sehr vielseitig, und im Stande der Technik sind auch verschiedene andere Führungsmandrins bekanntgeworden.

Ein weiterer Führungsmandrin, der sog. Nessler-Mandrin, ist aus der DE-OS 31 09 402 bekannt. Dieser Führungsmandrin besteht aus einem dünnen Stahldraht, der mit Kunststoff überzogen ist. Der Kunststoff überragt das proximale Ende des Stahldrahtes und bildet somit eine flexible Spitze. Dieser Mandrin kann bei der Venenpunktion zur Anlage von zentralen Venenkathetern verwendet werden. Auch bei diesem Mandrin tritt der Nachteil auf, daß er am proximalen Ende abknicken kann, da sich auch hier am proximalen Ende des Stahldrahtes die Stabilität und Flexibilität abrupt ändern. Der über den Stahldraht vorstehende Kunststoff muß daher eine ausreichende Stabilität haben, die jedoch beim Einlegen des Mandrins eine Gefäßperforation zur Folge haben kann.

Der Erfindung liegt die Aufgabe zugrunde, den Führungsmandrin der eingangs genannten Gattung so auszubilden, daß er eine hohe Flexibilität hat, die auch geringe Biegeradien zuläßt, eine ausreichende Stabilität aufweist, um ohne Verformung vorgeschoben werden zu können, aus einem Material besteht, das beim Zurückziehen durch Punktionskanülen nicht abgeschert wird, eine möglichst hohe Knickfestigkeit aufweist und außerdem als Massenprodukt herstellbar ist.

Weiterhin liegt der Erfindung die Aufgabe zugrunde, ein Verfahren zur Herstellung eines solchen Führungsmandrins zu schaffen.

Die Lösung der Aufgabe ist ausgehend von einem Führungsmandrin der eingangs als bekannt vorausgesetzten Art im Anspruch 1 gekennzeichnet.

Zweckmäßige Ausgestaltungen der Erfindung und ein vorteilhaftes Verfahren zu seiner Herstellung ergeben sich aus den Unteransprüchen.

Der Führungsmandrin gemäß Anspruch 1, der nach Art einer Litze hergestellt ist, hat eine hohe Flexibilität und ermöglicht eine Verformung auch bei sehr kleinen Biegeradien, hat aber gleichzeitig eine ausreichende Stabilität beim Vorschieben. Ein wesentlicher Vorteil liegt darin, daß sich am dem patienten zugekehrten seele keine abrupte Stabilitäts- und Flexibilitätsänderung ergibt, sondern die Größen sich kontinuierlich ändern und dadurch ein Abknicken in diesem Bereich vermieden werden kann. Durch entsprechende Materialwahl und Dimensionierung der Drahtseele und der den Mantel bildenden Einzeldrähte ist es praktisch möglich, jede gewünschte Flexibilität herzustellen.

Gemäß einer bevorzugten Ausführungsform nach Anspruch 2 besteht die Drahtseele selbst aus Einzeldrähten, die schrauben linienförmig gewik-

kelt, nebeneinanderliegend und flach geneigt zur Mandrinachse verlaufen. Dies gestattet Verformbarkeit auch bei sehr kleinen Radien. Von besonderem Vorteil ist auch das Merkmal des Anspruchs 3, wonach sich zwischen dem Mantel und der Drahtseele eine Kunststoff-Zwischenschicht befindet.

Zur Bildung einer glatten Oberfläche kann der Mantel mit einer dünnen Kunststoffschicht überzogen werden. Hierbei kann ein weicher Kunststoff verwendet werden, der Stabilität und Flexibilität des Mandrins nicht beeinflußt, da diese Größen durch die Drahtseele und den Mantel bestimmt werden. Gemäß Anspruch 4 bildet die Kunststoffschicht vorteilhafterweise eine Abschlußkappe.

Die Erfindung wird nachstehend anhand der Fig. 1 bis 3 beispielsweise erläutert. Es zeigt:

Fig. 1 einen Längsschnitt eines Führungsmandrins,

Fig. 2 und 3 Zwischenprodukte bei der Herstellung des Mandrins der Fig. 1.

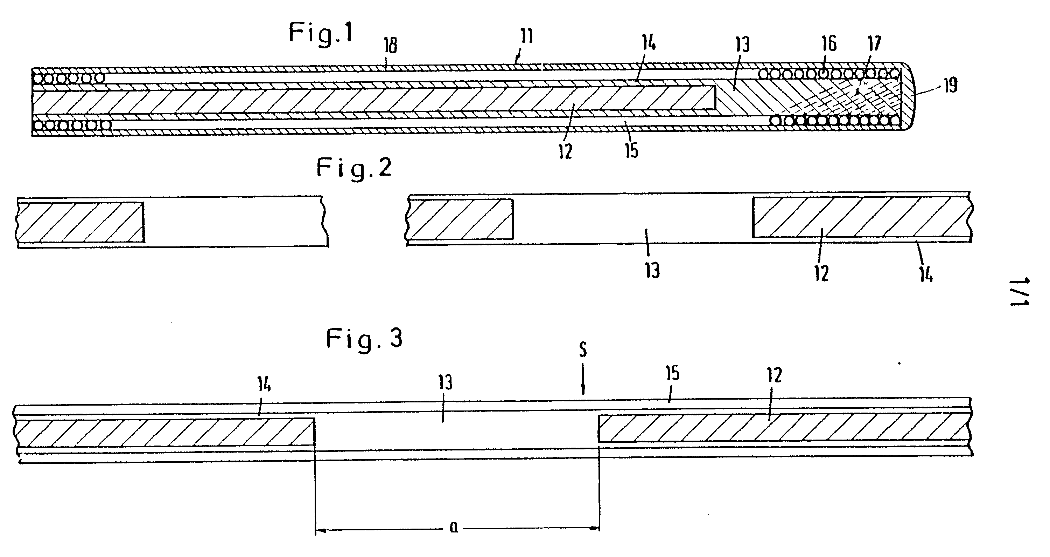

Fig. 1 zeigt einen Führungsmandrin 11, der eine Drahtseele 12 aufweist, die von einem Mantel 15 umgeben ist, der aus schematisch angedeuteten Einzeldrähten 16 besteht. Die Einzeldrähte sind, wie bei 17 angegeben ist, spiralförmig um die Drahtseele 12 gewickelt, liegen nebeneinander und verlaufen flachgeneigt zur Mandrinachse. Zwischen dem Mantel 15 und der Drahtseele 12 befindet sich eine Kunststoffzwischenschicht 14. Da das proximale Ende der Drahtseele 12 gegenüber dem proximalen Enden des Mantels 15 zurückgesetzt ist. ist auch der zwischen diesen Enden liegende Raum durch ein Kunststoffendstück 13 gefüllt.

Der Mantel 15 ist mit einer Kunststoffaußenschicht 18 überzogen. Außerdem ist das proximale Ende des Mandrins 11 mit einer Abschlußkappe 19 aus Kunststoff versehen.

Bei einem üblichen Außendurchmesser eines derartigen Führungsmandrins von z.B. 0,5 mm sollte die Drahtseele 12 einen Durchmesser von etwa 0,24 mm aufweisen. Nach Einbettung in Kunststoff beträgt der Gesamtdurchmesser der Drahtseele 12 0,28 mm. Der Mantel kann aus elf Einzeldrähten 16 mit jeweils einem Durchmesser von 0,09 mm bestehen, so daß sich ein Durchmesser des unbeschichteten Mandrins von etwa 0,47 mm und beschichtet mit einer dünnen Teflonschicht von, wie bereits erwähnt, etwa 0,5 mm ergibt. Die Drahtseele 12 kann selbst entsprechend dem Mantel 15 aus Einzeldrähten bestehen, z.B. aus sieben Einzeldrähten von je 0,09 mm Durchmesser, die nach Art einer Litzeneinlage ausgebildet sind.

Bei einem Mandrin mit Einzeldrahtseele sollte diese gegenüber dem proximalen Ende des Mantels 15 um etwa 40 mm zurückgesetzt sein. Die Dicke der Abschlußkappe 19 kann etwa 5 mm betragen.

Zur Herstellung eines Mandrins entsprechend Fig. 1 kann von einem Zwischenprodukt entsprechend Fig. 2 ausgegangen werden. Dieses Zwischenprodukt entsteht dadurch, daß einzelne Drahtseelen 12 entsprechend der gewünschten Mandrinlänge im Extrusionsverfahren mit weichem Kunststoff überzogen werden, so daß die Zwischenschicht 14 gebildet wird. Gleichzeitig mit der Bildung der Zwischenschicht können auch die Endstücke 13 extrudiert werden, die beim Zwischenprodukt Zwischenstücke zwischen den einzelnen Drahtseelen 12 bilden. Der Durchmesser der Endstücke 13 und der beschichteten Drahtseelen 12 sollte gleich sein.

Das Zwischenprodukt der Fig. 2 kann auf einer üblichen Maschine, wie sie zum Herstellen von Drahtseilen verwendet wird, mit dem Mantel 15 versehen werden, der durch Verdrillen der Einzeldrähte 16 entsteht, so daß sich das Zwischenprodukt der Fig. 3 ergibt. Der Abstand a zwischen den einzelnen Drahtseelen kann dabei etwa 40 mm betragen.

Das Zwischenprodukt der Fig. 3 Kann nun noch mit der Außenschicht 18 versehen werden. Die einzelnen Führungsmandrine werden dadurch fertiggestellt, daß das Zwischenprodukt der Fig. 3 etwa an der durch den Pfeil S angegebenen Stelle geschnitten wird. Anschließend ist es noch möglich, die Abschlußkappe 19 anzuformen.

Führungsmandrine, die ohne eine Kunststoffaußenschicht hergestellt werden, können an der Spitze durch Verzinnen oder Laser-Punktschweißen entgratet werden, um vor Verletzungen zu schützen. Die Kunststoffaußenschicht ist insbesondere in Verbindung mit der Abschlußkappe von Vorteil, da sie verhindert, daß sich die Einzeldrähte des Mantels lösen. Außerdem sichert eine derartige Schicht eine gleichmäßige Außenfläche des Mandrins.

Für die Herstellung der Drahtseele und des Mantels kann ein Federstahldraht mit einer Nennfestigkeit von 1570 N/mm$^2$ verwendet werden, wie er üblicherweise auch zur Herstellung von Drahtseilen zur Anwendung gelangt. Als Kunststoffmaterial eignet sich jedes ausreichend flexible Material, da Stabilität und Flexibilität des Mandrins auch am proximalen Ende in erster Linie durch die Drahtseele und/oder den umgebenden Mantel gewährleistet sind.

Selbstverständlich ist es auch möglich, zur Herstellung solcher Führungsmandrine einen durchgehenden Kerndraht zu verwenden, der nach der Bildung dem Mantels entsprechend der gewünschten Länge eines fertigen Führungsmandrins geschnitten wird. Anschließend ist es dann noch erforderlich, die Drahtseele gegenüber dem Mantel zurückzuziehen, damit dieser am proximalen Ende über die Drahtseele vorsteht.

Bei der Herstellung des Mantels und auch einer nach Art einer Litzeneinlage ausgebildeten Drahtseele kann es von besonderer Bedeutung sein, die Einzeldrähte spannungsarm schraubenlinienförmig zu wickeln. Hierzu wird den Einzeldrähten die Schraubenlinienform schon vor dem Wikkeln erteilt, wie dies bei der Herstellung eines TRU-LAY-Seils bekannt ist. Diese Maßnahme verhindert das Auftreten von Spannungen in den Einzeldrähten und erhöht damit die Flexibilität des Mandrins. Außerdem verhindert diese Maßnahme, daß sich der Mantel insbesondere am proximalen Mandrinende aufspleißt.

## Ansprüche

1. Führungsmandrin für medizinische Katheter und dgl. mit einer Drahtseele, die von einem geschlossenen Mantel aus mehreren, mit flacher Neigung zur Mandrinachse schraubenlinienförmig gewickelten Drähten umgeben und die am dem patienten zugekehrten Mandrinende gegenüber dem Mantel zurückgesetzt ist, **dadurch gekennzeichnet,** daß die Einzeldrähte des Mantels nebeneinander liegen und aus Federstahlmaterial bestehen und daß die Drahtseele auch aus Federstahlmaterial besteht.

2. Führungsmandrin nach ANspruch 1, **dadurch gekennzeichnet** daß die Drahtseele selbst aus Einzeldrähten besteht, die mit flacher Neigung zur Mandrinachse schraubenlinienförmig, nebeneinanderliegend gewickelt sind.

3. Führungsmandrin nach Anspruch 1 oder 2, **dadurch gekennzeichnet,** daß sich zwischen dem Mantel (15) und der Drahtseele (12) eine Kunststoffzwischenschicht (14) befindet.

4. Führungsmandrin nach Anspruch 1, 2 oder 3, **dadurch gekennzeichnet,** daß der Raum zwischen dem zurückgesetzten Ende der Drahtseele (12) und dem Mandrinende mit Kunststoff gefüllt ist.

5. Führungsmandrin nach einem der Ansprüche 1 bis 4, **dadurch gekennzeichnet,** daß der Mantel (15) mit einer Kunststoff-Aussenschicht umgeben ist, die am dem Patienten zugekehrten Einführende eine Abschlußkappe (19) bildet.

6. Führungsmandrin nach einem der Ansprüche 1 bis 5, **dadurch gekennzeichnet,** daß bei einem Durchmesser des Mantels (15) von etwa 0,47 mm die Drahtseele (12) gegenüber dem Einführende des Mantels (15) um etwa 30 bis 60 mm zurückgesetzt ist.

7. Führungsmandrin nach Anspruch 6, **dadurch gekennzeichnet,** daß der Durchmesser der Drahtseele (12) etwa 0,24 mm und mit Kunststoff beschichtet 0,28 mm beträgt.

8. Führungsmandrin nach einem der Ansprüche 1 bis 7, **dadurch gekennzeichnet,** daß der Mantel ( 15) aus elf Einzeldrähten (16) mit einem Durchmesser von etwa 0,09 mm besteht.

9. Führungsmandrin nach einem der Ansprüche 1 bis 8, **dadurch gekennzeichnet,** daß die Kunststoff-Aussenschicht (18) eine Dicke von etwa 0,03 mm hat.

10. Führungsmandrin nach einem der Ansprüche 1 bis 9, **dadurch gekennzeichnet,** daß die Einzeldrähte der Drahtseele wenigstens einen zentralen Einzeldraht umgeben.

11. Führungsmandrin nach Anspruch 10, **dadurch gekennzeichnet,** daß die Drahtseele (12) aus sieben Einzeldrähten mit einem Durchmesser von jeweils etwa 0,09 mm besteht.

12. Verfahren zur Herstellung des Führungsmandrins nach einem der Ansprüche 3 bis 11, **gekennzeichnet** durch folgende Verfahrensschritte:
    a) aufeinanderfolgende, beabstandete Drahtseelen werden durch Extrusion mit Kunststoff beschichtet;
    b) gleichzeitig werden die Zwischenräume zwischen den einzelnen Drahtseelen mit Zwischenstücke bildendem Kunststoff gefüllt;
    c) wobei die Auffüllung der Zwischenstücke bis zu dem gleichen Aussendurchmesser wie dem der Kunststoffbe schichtung der Drahtseelen erfolgt;
    d) die beschichteten Drahtseelen und die Zwischenstücke werden zur Bildung des Mantels mit Einzeldrähten schraubenlinienförmig umwickelt;
    e) die zusammenhängenden und mit dem Mantel versehenen Drahtseelen werden an einem Ende geschnitten.

## Claims

1. Guide mandrel for medical catheters and the like, having a wire core which is surrounded by a closed sleeve of a plurality of wires which are wound helically at a shallow inclination to the mandrel axis and the end of the mandrel close to the patient is set back with respect to the sleeve, characterised in that the individual wires of the sleeve lie side by side and comprise spring steel material, and the wire core also comprises spring steal material.

2. Guide mandrel as in claim 1, characterised in that the wire core itself comprises individual wires, which are wound helically side by side at a shallow inclination to the mandrel axis.

3. Guide mandrel as in claim 1 or 2, characterised in that an intermediate plastics layer is located between the sleeve (15) and the wire core (12).

4. Guide mandrel as in claim 1, 2 or 3, characterised in that the space between the set-back end of the wire core (12) and the end of the mandrel is filled with plastics material.

5. Guide mandrel as in any Of claims 1 to 4, characterised in that the sleeve (15) is surrounded by an outer plastics layer, which forms a cap (19) on the insertion end close to the patient.

6. Guide mandrel as in claims 1 to 5, characterised in that, given a diameter of the sleeve (15) of around 0.47 mm, the wire core (12) is set back with respect to the insertion end of the sleeve (15) by around 30 to 60 mm.

7. Guide mandrel as in claim 6, characterised in that the diameter of the wire core (12) is around 0.24 mm and, coated in plastics material, is 0.28 mm.

8. Guide mandrel as in any of claims 1 to 7, characterised in that the sleeve (15) comprises eleven individual wires (16) having a diameter of around 0.09 mm.

9. Guide mandrel as in any of claims 1 to 8, characterised in that the outer plastics layer (18) has a thickness of around 0.03 mm.

10. Guide mandrel as in any of claims 1 to 9, characterised in that the individual wires of the wire core surround at least one central individual wire.

11. Guide mandrel as in claim 10, characterised in that the wire core (12) comprises seven individual wires each having a diameter of around 0.09 mm.

12. Method of making the guide mandrel as in any of claims 3 to 11, characterised by the following method stages:
    a) successive spaced-apart wire cores are extrusion-coated with plastics;
    b) at the same time the spaces between the individual wire cores are filled with plastics which forms spacers;
    c) wherein the spacers are filled up to the same outer diameter as that of the plastics coating;
    d) the coated wire cores and the spacers are wound helically with individual wires to form the sleeve;
    e) the wire cores, which are connected and are provided with the sleeve, are cut at one end.

**Revendications**

1. Mandrin de guidage pour cathéter à usage médical ou analogue, comportant une âme constituée d'un fil qui est entouré par une gaine fermée constituée de plusieurs fils enroulés en hélice avec une faible pente par rapport à l'axe du mandrin et l'extrémité du mandrin tournée vers le patient est en retrait par rapport à la gaine, caractérisé en ce que les fils séparés constituant la gaine sont juxtaposés et sont en acier à ressort et le fil constituant l'âme est également an acier à ressort.

2. Mandrin de guidage selon la revendication 1, caractérisé en ce que le câble formant l'âme est lui-même formé de fils séparés qui sont enroulés de manière jointive, en hélice, avec une faible pente par rapport à l'axe du mandrin.

3. Mandrin de guidage selon la revendication 1 ou 2, caractérisé par une couche intermédiaire en matière synthétique (13) entre la gaine (15) et le câble formant l'âme (12).

4. Mandrin de guidage selon la revendication 1, 2 ou 3, caractérisé en ce que l'intervalle entre l'extrémité en retrait du câble formant l'âme (12) et l'extrémité du mandrin est rempli de matière synthétique.

5. Mandrin de guidage selon l'une des revendications 1 à 4, caractérisé en ce que la gaine (15) est entourée d'une couche extérieure en matière synthétique qui forme un capuchon d'obturation (19) à l'extrémité d'introduction située du côté du patient.

6. Mandrin de guidage selon l'une des revendications 1 à 5, caractérisé en ce que pour un diamètre de la gaine (15) de l'ordre de 0,47 mm, le câble formant l'âme (12) est en retrait d'environ 30 à 60 mm par rapport à l'extrémité d'introduction de la gaine (15).

7. Mandrin de guidage selon la revendication 6, caractérisé en ce que le diamètre du câble formant l'âme (12) est de l'ordre de 0,24 mm et est revêtu d'une matière synthétique sur 0,28 mm.

8. Mandrin de guidage selon l'une des revendications 1 à 7, caractérisé en ce que la gaine (15) est formée de onze fils séparés (16) ayant chacun un diamètre d'environ 0,09 mm.

9. Mandrin de guidage selon l'une des revendications 1 à 8, caractérisé en ce que la couche extérieure en matière synthétique (18) a une épaisseur de l'ordre de 0,03 mm.

10. Mandrin de guidage selon l'une des revendications 1 à 9, caractérisé en ce que les fils séparés formant le câble de l'âme entourent au moins un fil central séparé.

11. Mandrin de guidage selon la revendication 10, caractérisé en ce que le câble formant l'âme (12) est constitué de sept fils séparés ayant chacun un diamètre de l'ordre de 0,09 mm.

12. Procédé pour la réalisation d'un mandrin de guidage selon l'une des revendications 3 à 11, caractérisé en ce que :

    a) on réalise un revêtement en matière synthétique par extrusion sur les câbles formant l'âme, successifs, distants les uns des autres,

    b) en même temps on remplit les intervalles des câbles séparés formant les âmes avec de la matière synthétique formant des pièces intermédiaires,

    c) le remplissage des pièces intermédiaires se fait jusqu'au même diamètre extérieur que le revêtement de matière synthétique des câbles formant les âmes,

    d) les âmes de câbles revêtues et les pièces intermédiaires sont enroulées en hélice avec les fils séparés pour former la gaine,

    e) on coupe une extrémité des différents câbles formant les âmes, reliés entre eux et munis de la gaine.

Fig.1

Fig.2

Fig.3